# EUROPEAN PATENT SPECIFICATION

(11) **EP 3 983 798 B1**
(45) Date of publication and mention of the grant of the patent: **26.06.2024**
(21) Application number: 20758363.4
(22) Date of filing: 09.06.2020
(51) Int. Cl.: G01N 33/04, A01J 5/007

(54) **MILK ANALYSIS SYSTEM**
MILCHANALYSESYSTEM
SYSTEME D'ANALYSE DE LAIT

(30) Priority: 14.06.2019 SE 1950717
(43) Date of publication of application: 20.04.2022
(73) Proprietor: DeLaval Holding AB, 147 21 Tumba (SE)
(72) Inventor: DALLERUP RASMUSSEN, Claus, 147 21 Tumba (SE); SLAABY, John, 147 21 Tumba (SE)
(74) Representative: DeLaval International AB
(86) International application number: PCT/SE2020/050584
(87) International publication number: WO 2020/251458

(56) References cited:
- WO-A1-2018/236271
- CN-U- 208 624 367
- US-A1- 2016 082 165

## Description

### TECHNICAL FIELD

This document discloses a system comprising a dosing module, insertable into a milk analysis apparatus, and a control unit of the milk analysis apparatus. More particularly, it is herein presented a control unit of a milk analysis apparatus comprising a first wireless communication device, for communication with a memory device of the dosing module, which dosing module is insertable into the milk analysis apparatus and comprises a pump, via a second wireless communication device comprised in the dosing module.

### BACKGROUND

On an animal farm, it is important to keep the animals healthy in order to enhance milk/ meat production, and/ or to monitor when animals are in heat and/ or pregnant, for example. It is important to inseminate animals at an optimal moment in order to successfully fertilise the animal. In case the animal is not successfully inseminated, milk production is affected.

Several biomarker measurements may be made on the animal, such as e.g. measuring levels of progesterone, LDH (Lactate Dehydrogenase), BHB (Beta-Hydroxybutyrat) and urea. Thereby important information concerning e.g. heat detection and/ or pregnancy of the individual animal may be made (based on measured progesterone level), as well as mastitis (based on LDH) and ketosis (based on BHB). Also, the energy balance of the animal may be estimated (based on urea). One example of an arrangement configured to measure a biomarker value of an animal milk sample is described in document WO2018/236271 A1. The arrangement described therein comprises a cassette which comprises a carrier tape comprising test zones for indicating the biomarker value. The carrier tape is arranged on spools arranged to co-operate with a cassette external motor.

Thereby, a farmer/ operator is provided with important information concerning status of each individual animal. However, to perform and analyse biomarker measurements of all individual animals at a farm, e.g. by applying milk samples on prepared dry sticks, and analyse these samples are time consuming for the farmer, who may have to take care of various other important issues. It also put high demands on administrative skills on the farmer to distinguish biomarker measurements from different animals; to keep track on when it is time to repeat the biomarker measurement for each individual animal and when to change biomarker measurement units; maintenance of the biomarker test equipment as well as high demands on cleanliness for not allowing a biomarker measurement of a first animal to be contaminated by biological matters of another animal.

A milk analysis apparatus may be arranged to cooperate with a milk extracting arrangement, for regularly analysing milk samples of the animals, e.g. at or around the moment of a milking session. The milk analysis apparatus may extract a milk sample and provide it on a milk analysis unit such as a dry stick/ lateral flow stick! lateral flow test strip or similar. The milk is typically diluted with a diluent, which also may be used to rinse the tubings between test sessions. The diluent may be provided in a liquid container.

The tubing/s and pump/s for propagating the milk and/ or the dilutant may age and wear over time and may have to be exchanged at certain intervals, dependent on running time of the pump/s and/ or exposure time of milk to the tubing/s. The tubings or hoses are connected to a milking extracting machine such as a milking robot and also to the liquid container. The thin hoses will be repeatedly squeezed by the pump to forward the milk and diluent to the dosing needle. There is a risk that the hoses will not flex back to their original shape after a certain period of usage. The lifetime for the dosing module may be approximated to about 6 to 8 months and thereafter it has to be exchanged. However, the tubing/s and/ or pump/s of different milk analysis apparatus on the farm may have to be changed at different points in time, leading to an administrative inferno for the farmer.

It would be desired to find a solution for assisting the farmer in keeping track on usage of the tubings/ pumps on the farm in different milk analysis apparatus, and to assist him/ her in changing them in due time for both avoiding the tubings and/ or pump becomes too deteriorated to function, i.e. start to clog or leak and/ or become too stiff to efficiently propagate the milk/ diluent; while at the same time not changing the tubings and/ or pump too early which would be an unnecessary waste of resources.

WO2018/236271 discloses a system comprising a control unit, a milk analysis apparatus, a wireless communication device and a dosing module with a pump.

### SUMMARY

It is therefore an object of this invention to solve at least some of the above problems and facilitate for a farmer to measure a biomarker value of a milk sample of an animal.

A biomarker, or biological marker, generally refers to a measurable indicator of some biological state or condition of the animal. The biomarker value measurement may be associated with pregnancy/ reproduction of the animal, health of the animal, and/ or quality of the milk of the animal.

According to the invention, this objective is achieved by a system comprising a control unit of a milk analysis apparatus, in an agricultural environment. The milk analysis apparatus comprises a first wireless communication device, for communication with a memory device of a dosing module. The dosing module is insertable into the milk analysis apparatus. Further, the dosing module comprises a second wireless communication device for communication with the first wireless communication device of the milk analysis apparatus. The dosing module comprises one or several pumps. The control unit is configured to determine a measurement relating to usage of the pump. Further, the control unit is configured to transmit information concerning the determined measurement to the memory device of the dosing module, via the first wireless communication device, for storage in the memory device of the dosing module.

In a first possible implementation of the control unit wherein the measurement relating to usage of the pump comprises usage time, the control unit may be configured to detect when the pump of the dosing module is used for the first time. The control unit may also be configured to determine a moment in time when the first-time usage of the pump is detected. In addition, the control unit may be configured to transmit the determined moment in time to the memory device of the dosing module, via the first wireless communication device, for storage in the memory device of the dosing module.

In a second possible implementation of the control unit the control unit may be configured to determine a current moment in time; and to check whether the past time between the stored moment in time of the memory device of the dosing module, associated with the first-time usage of the pump, and the determined current moment in time exceeds a predetermined time limit. The control unit may also be configured to discontinue usage of the dosing module when the predetermined time limit is exceeded.

In a third possible implementation of the control unit wherein the measurement relating to usage of the pump comprises a time gap of inactivity of the pump, the control unit may be configured to obtain a time value indicating when the pump was last used, from the memory device of the dosing module, via the first wireless communication device. Also, the control unit may be configured to determine a current moment in time. The control unit may in addition be configured to check whether the past time between the current moment in time and the time value of the last pump usage exceeds a predetermined inactivity time limit. Furthermore, the control unit may be configured to discontinue usage of the dosing module when the predetermined inactivity time limit is exceeded.

In a fourth possible implementation of the control unit wherein the measurement relating to usage of the pump comprises pumped amount of liquid by the pump, the control unit may be configured to obtain a value indicating amount of pumped liquid made by the pump, from the memory device of the dosing module, via the first wireless communication device in case the pump has been previously used. In addition, the control unit may be configured to determine amount of pumped liquid by the pump during a test session. The control unit may also be configured to calculate an amount of totally pumped liquid by the pump by adding the amount of liquid pumped during the test session to the obtained amount of previously pumped liquid. Furthermore, the control unit may be configured to transmit the calculated amount of totally pumped liquid by the pump to the memory device of the dosing module, via the first wireless communication device, for storage in the memory device of the dosing module.

In a fifth possible implementation of the control unit the control unit may be configured to check whether the calculated amount of totally pumped liquid by the pump exceeds a predetermined volume limit. The control unit may also be configured to discontinue usage of the dosing module when the predetermined volume limit is exceeded.

In a sixth possible implementation of the control unit wherein the measurement relating to usage of the pump comprises running time of the pump, the control unit may be configured to estimate a total running time of the pump. The control unit may also be configured to compare the total running time of the pump with a running time threshold limit. Additionally, the control unit may be configured to discontinue usage of the dosing module when the running time threshold limit is exceeded.

In a seventh possible implementation of the control unit the control unit may be configured to output an alert, encouraging an agricultural manager to change the dosing module when any of the predetermined time limit, the predetermined inactivity time limit, the predetermined volume limit, and/ or the running time threshold limit is exceeded, via an output device.

In an eighth possible implementation of the control unit the control unit may be configured to activate the first wireless communication device when starting a liquid extraction session with the pump; and/ or deactivate the first wireless communication device when the information concerning the determined measurement to the memory device of the dosing module has been transmitted.

According to the invention, the system further comprises a dosing module comprising a pump, a memory device and a second wireless communication device. The dosing module is insertable into a milk analysis apparatus. The dosing module is intended for information exchange with a control unit of the milk analysis apparatus via a first wireless communication device of the milk analysis apparatus. The memory device of the dosing module is configured to receive information concerning a measurement relating to usage of the pump from the control unit of the milk analysis apparatus, via the second wireless communication device. The memory device is also configured to store the received information.

In a first possible implementation of the dosing module wherein the memory device comprises an indicator of virginity when the pump of the dosing module is unused, the memory device may be configured to provide the indicator of virginity to the control unit of the milk analysis apparatus, via the second wireless communication device, when the pump is used for the first time.

In a second possible implementation of the dosing module the memory device may be configured to receive information concerning a moment in time of a usage of the pump from the control unit of the milk analysis apparatus, via the second wireless communication device. In addition, the memory device may also be configured to store the received information.

In a third possible implementation of the dosing module the memory device may be configured to provide stored information concerning a moment in time of a first-time usage of the pump to the control unit of the milk analysis apparatus, via the second wireless communication device.

In a fourth possible implementation of the dosing module the memory device may be configured to provide stored information concerning a time value indicating when the pump was last used, to the control unit of the milk analysis apparatus, via the second wireless communication device.

In a fifth possible implementation of the dosing module the memory device may be configured to provide a value indicating amount of pumped liquid made by the pump, to the control unit of the milk analysis apparatus, via the second wireless communication device. In further addition, the memory device may be configured to obtain an updated value indicating amount of pumped liquid made by the pump from the control unit of the milk analysis apparatus, via the second wireless communication device. The memory device may also be configured to store the received information.

In a sixth possible implementation of the dosing module the memory device may be configured to receive a blocking sign prohibiting further usage of the dosing module from the control unit via the second wireless communication device. Also, the memory device may be additionally configured to store the received blocking sign, thereby prohibiting further usage of the dosing module.

Thus, the system according to the invention comprises the control unit, the dosing module and also a milk analysis apparatus. The dosing module comprises the memory device and the second wireless communication device, which dosing module is insertable into the milk analysis apparatus, for information exchange with the control unit of the milk analysis apparatus via a first wireless communication device of the milk analysis apparatus; wherein the memory device is configured to:
receive information concerning a measurement relating to usage of the pump from the control unit of the milk analysis apparatus, via the second wireless communication device; and
store the received information; wherein
   the milk analysis apparatus comprises the first wireless communication device, for communication with the memory device of the dosing module, via the second wireless communication device comprised in the dosing module, and wherein
   the control unit is configured to:
      determine a measurement relating to usage of the pump; and
      transmit information concerning the determined measurement to the memory device of the dosing module, via the first wireless communication device, for storage in the memory device of the dosing module.

The system also comprises a milk extracting arrangement, operating in conjunction with the milk analysis apparatus.

Thereby, by keeping track of the operational usage of the dosing module and the components comprised therein during a liquid extraction session and repeatedly store this information in the memory device of the dosing module, it may be estimated if/ when it is time to change the dosing module, also in case the dosing module is inserted into another milk analysis apparatus. Replacement of dosing modules in due time is thereby ascertained. It also becomes possible to trace the history of the dosing module, e.g. in case of malfunction or reclamation of the milk analysis apparatus. Non-authorised reusage of the dosing modules is also avoided.

It is hereby avoided that a liquid extraction session is initiated with a dosing module being deteriorated due to excessive usage and/ or aging components. Further, measures may be taken to ascertain that a new dosing module is ordered in time and that the dosing module is replaced at the right moment to avoid unnecessary waste of dosing module components, while not disturbing the planned milk sample testing of the milk analysis apparatus. Hereby costs, maintenance and work intensity of the farmer associated with management of the milk analysis apparatus is minimised or at least reduced.

Other advantages and additional novel features will become apparent from the subsequent detailed description.

### FIGURES

Embodiments of the invention will now be described in further detail with reference to the accompanying figures, in which:
- **Figure 1**: illustrates an example of an arrangement for measuring a biomarker value of a milk sample of an animal.
- **Figure 2A**: illustrates a cassette inserted into a milk analysis apparatus, according to an embodiment.
- **Figure 2B**: illustrates a section of a tape comprising dry sticks, according to an embodiment.
- **Figure 3A**: illustrates the cassette, a liquid container and the milk analysis apparatus, according to an embodiment.
- **Figure 3B**: illustrates the cassette from a side view.
- **Figure 4**: illustrates a first side of the milk analysis apparatus.
- **Figure 5**: illustrates a second side of the milk analysis apparatus.

### DETAILED DESCRIPTION

The invention may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

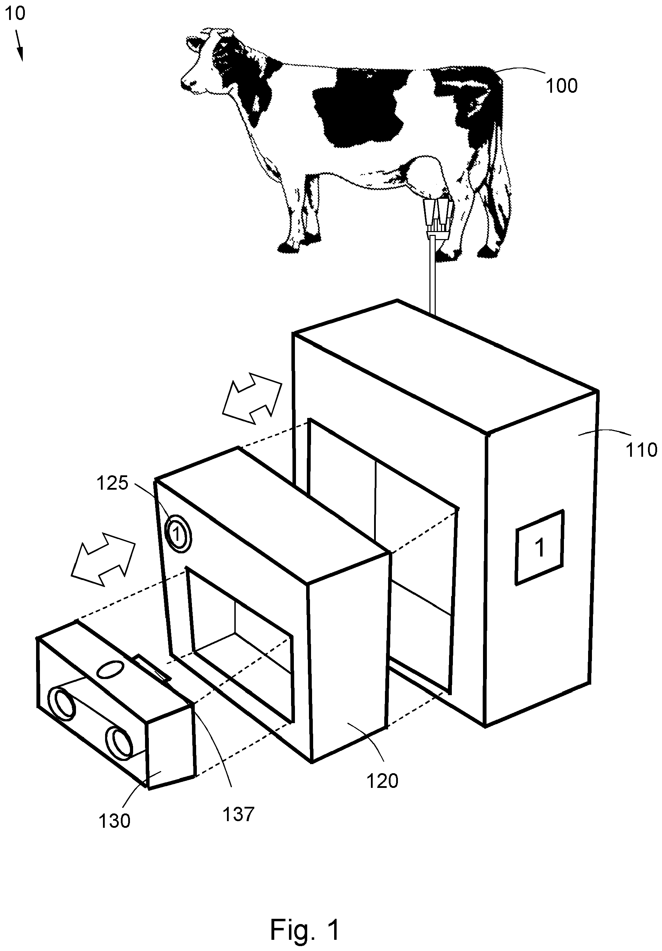

**Figure 1** illustrates a scenario with a system 10 for analysing milk of an animal 100 which may be comprised in a herd of dairy animals at a dairy farm.

"Animal" may be any arbitrary type of domesticated female milk producing and/ or meat producing mammal such as cow, goat, sheep, horse, camel, primate, dairy buffalo, donkey, yak, etc.

Milk of the animal 100 may be extracted by a milk extracting arrangement, or milking equipment 110 such as e.g. a milking robot or other milking arrangement and provided to a milk analysis apparatus 120.

The milk analysis apparatus 120 may be associated with and possibly even releasably insertable into the milk extracting arrangement 110 in some embodiments. Thus, there may be an interface between the milk extracting arrangement 110 and the milk analysis apparatus 120 for providing milk and possibly also electricity via the milk extracting arrangement 110 to the milk analysis apparatus 120.

The milk analysis apparatus 120 comprises various electronics and equipment such as a camera, one or several pumps, a tube element for attachment to the interface to the milk extracting arrangement 110, motors, a communication unit etc.

A cassette 130 may be detachably inserted into the milk analysis apparatus 120. The cassette 130 may comprises a tape or similar corresponding arrangement with milk analysis units such as dry sticks/ lateral flow sticks/ lateral flow test strips or similar. The milk analysis units are configured to indicate a biomarker value of a milk sample of the animal 100, e.g. indicate progesterone in the milk sample by a lateral flow test.

The cassette 130 may in some embodiments be configured to be detachably inserted in the milk analysis apparatus 120 and held in place by a fastening means such as a snap lock, a magnet, a screw, etc., and a door of the milk analysis apparatus 120 may be closed for enclosing the cassette 130 within the milk analysis apparatus 120, thereby further fixating the cassette 130 in the position.

Also, a liquid container may be insertable into, or associated with (i.e. physically connected to) the milk analysis apparatus 120. The liquid container may comprise a diluent, which may be used to dilute the extracted milk and also, or alternatively, to rinse the tubing, needle, pump etc., of the milk analysis apparatus 120.

The tubing, needle, pump etc. may be kept in a dosing module 137. Thereby, the tubing, needle, and pump may be replaced all at the same time in one single replacement.

Thereby, a milk sample of the animal 100 may be extracted from the animal 100 by the milk extracting arrangement 110 and provided via the milk analysis apparatus 120 to one of the milk analysis units on the tape of the cassette 130. The milk analysis units may react on presence and/ or amount of one or several biomarkers, e.g. by changing colours, or intensity of a colour. The camera in the milk analysis apparatus 120 may capture an image through an opening of the cassette 130. The captured image of the milk analysis unit may then be analysed by a control unit, and based on the intensity of the colour, presence and/ or quantity of the biomarker in the milk sample may thereby be determined.

The measured biomarker may be e.g. progesterone, glycoprotein, oestrogen and/ or Gon-adatropin-Releasing Hormones, or any other similar biomarker associated with reproduction or health of the animal 100, in different embodiments.

Progesterone is a hormone that regulates several physiological functions of the animal 100. Progesterone may prepare the uterus for pregnancy, maintain the pregnancy if fertilisation occurs, and inhibit the animal 100 from showing signs of standing oestrus and ovulating when pregnant. Progesterone levels, for example, may rise at the beginning of the pregnancy, and be kept at a high level throughout the pregnancy of the animal 100. Progesterone levels in milk samples may be used to monitor pregnancy, oestrous cycles (heat detection) and/ or postpartum ovarian activity. For these reasons, progesterone levels of animals 100 at the farm is interesting for the farmer to detect and keep track of.

However, the measured biomarker may in some embodiments comprise LDH (Lactate Dehydrogenase), BHB (Beta-HydroxyButyrat), urea, and/ or somatic cell count; or other biomarker related to status of the animal 100. In some embodiments, a plurality of the above enumerated biomarkers may be measured. Alternatively, in some embodiment, the farmer may subscribe to a cassette 130 comprising a certain milk analysis units configured to measure a biomarker, or a set of biomarkers, as selected by the farmer; and/ or different cassettes 130 comprising milk analysis units, e.g. on the tape configured to measure different biomarkers, or sets of biomarkers, during different periods of time of the year.

Thus, the milk analysis apparatus 120 comprises several modules such as the cassette 130, the dosing module 137 and/ or the liquid container, which are to be changed for a new respective module at particular time intervals, which may occur at different moments in time for the different modules; or alternatively synchronised with each other.

The cassette 130 with the milk analysis units is to be exchanged when the milk analysis units have been consumed. However, the number of used milk analysis units will be dependent on how often sampling is made, how many milk analysis units that are used for each animal 100 and/ or how many defect milk analysis units there are in the cassette 130. For example, in case the farmer is primarily interested in timing insemination of the animal 100, samples of that animal 100 may be taken only around, or right before, a moment in time when it is predicted that the particular animal 100 is in heat. Animals 100 such as cows and heifers typically go into heat or oestrus every 17 to 24 days (in average 21 days), why the next heat could be roughly predicted based on knowledge of the moment in time of the last heat.

Another farmer may want to take a milk sample on every milking event, e.g. in order to survey health status of a particular animal 100. Also, different cassettes 130 may possibly comprise a different number of milk analysis units. The time period between exchange of the cassettes 130, dosing modules 137 and/ or liquid container for different milk analysis apparatuses 120 may thereby be different.

The dosing module 137 comprises one or several pumps, such as hose pumps and a tube element for attachment to the milk extracting arrangement 110. The one or several pumps may be configured to act on the tube element for advancing the milk sample from the milk extracting arrangement 110 through the tube element to a needle. The milk sample may then be diluted with diluent from the liquid container in a mixing chamber of the needle (or possibly in a separate mixing chamber and then provided to the needle), where after the diluted milk sample may be applied from the needle to the milk analysis unit.

The pumps and the tubings of the dosing module 137 are typically made of plastic such as e.g. polyethylene, polypropylene, polyurethane, silicone; rubber, latex or similar material which will age and become fragile over time, also in case the dosing module 137 is not used. Also, milk may clog the tubings and affect the flow through the tubings. The milk may also stimulate aging of the tubings, so that it becomes stiff and fragile. For these reasons, the dosing module 137 may be exchanged for a new one either after a certain time period after first usage of the dosing module 137, a certain time period after production of the dosing module 137 and/ or when the pump has pumped a certain amount of milk through the tubings.

The liquid container comprises a diluent to be used as previously described. The liquid container has to be exchanged either when the diluent has been consumed, or after a certain time period exceeding a time threshold limit, when a chemical aging process may has caused the diluent to deteriorate.

The various modules, i.e. the dosing module 137, the liquid container and/ or the cassette 130 may comprise fastening means, e.g. in form of a snap fit arrangement, magnetics, screw joints, etc., arranged to attach the respective module onto the milk analysis apparatus 120.

The modular structure of the provided solution has several advantages. By keeping the arrangement modular in form of the dosing module 137, the liquid container and the cassette 130, which may be attached to milk extracting arrangement 110 of the farm; costs, maintenance and work intensity of the farmer may be minimised or at least reduced. Also, by separating the consumable material such as milk analysis units/ measurement sticks of the cassette 130, from elements subject to wear, like the pumps of the dosing module 137, and the electronics and instruments of the milk analysis apparatus; the liquid container could be continuously replaced with another replacement liquid container e.g. ordered via a courier service or postal office subscription.

The milk analysis apparatus 120 may on the other hand be detached from the milk extracting arrangement 110 and the dosing module 137/ liquid container/ cassette 130 upon malfunction and sent to a workshop for troubleshooting, repair, maintenance, etc.

Meanwhile, an identical replacement milk analysis apparatus 120 may be provided to the farm, enabling continuous biomarker measurements on the farm, also when the equipment of the milk analysis apparatus 120 is malfunctioning, to which the old dosing module 137/ liquid container/ cassette 130 could be applied.

Further, the arrangement may be operated by the farmer without requiring a particularly trained technician to come and visit the farm. Instead, the farmer may send the malfunctioning module to the workshop; or just replace it.

By maintaining the diluent, the milk analysis units and the tubings, respectively, in separate modules, they are protected from any possible affection of the environment at the farm.

However, an appearing problem is that it may become problematic for the farmer to keep track on which module to change at which moment in time. It would be desired to find a tool for reminding the farmer of when to change the respective modules, or at least one particular of the modules; and/ or to make it impossible to run the milk analysis apparatus 120 with clogged tubings of the dosing module 137, an empty liquid container, etc.

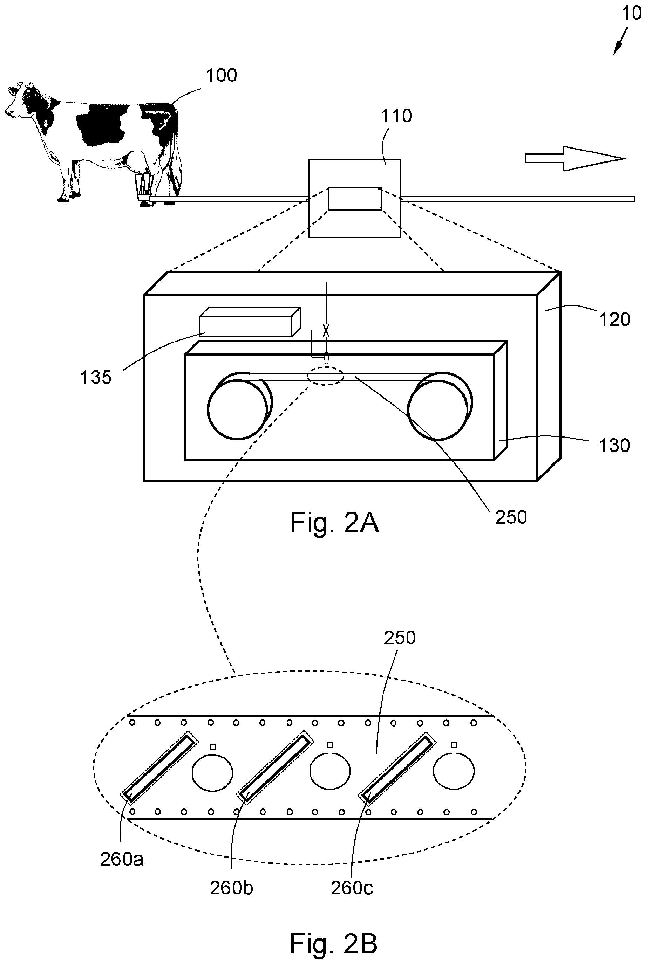

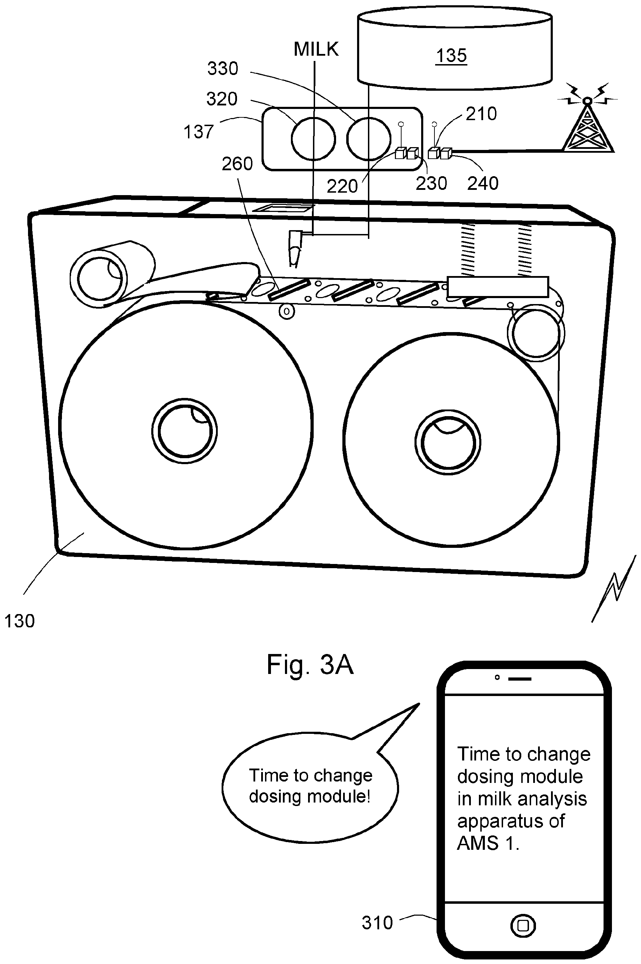

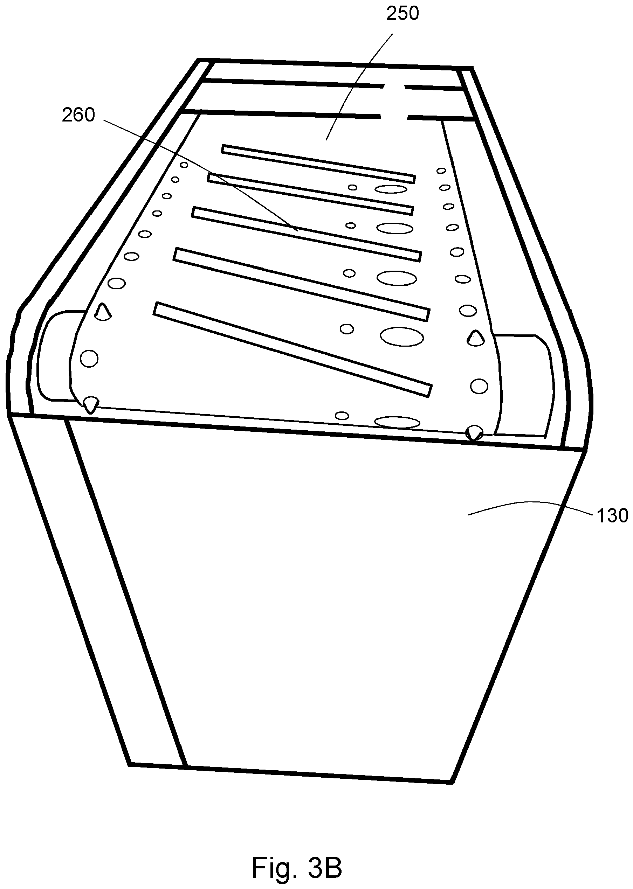

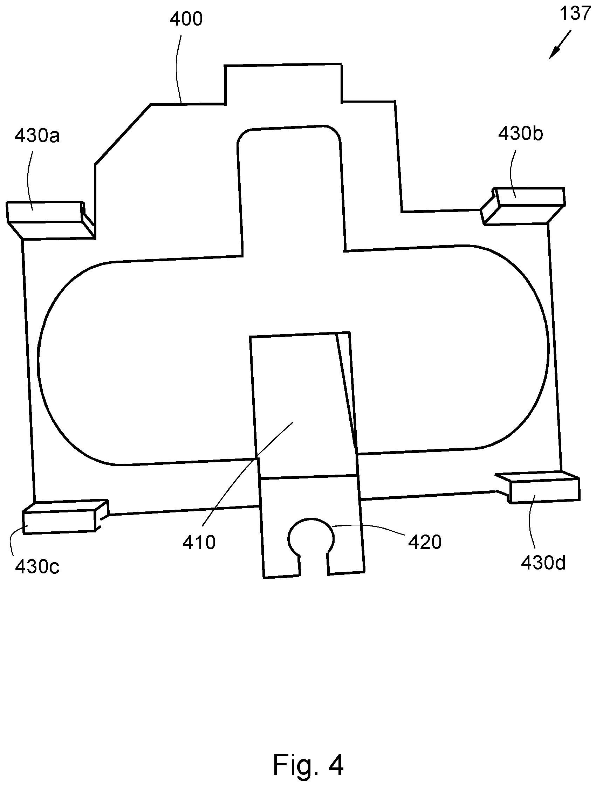

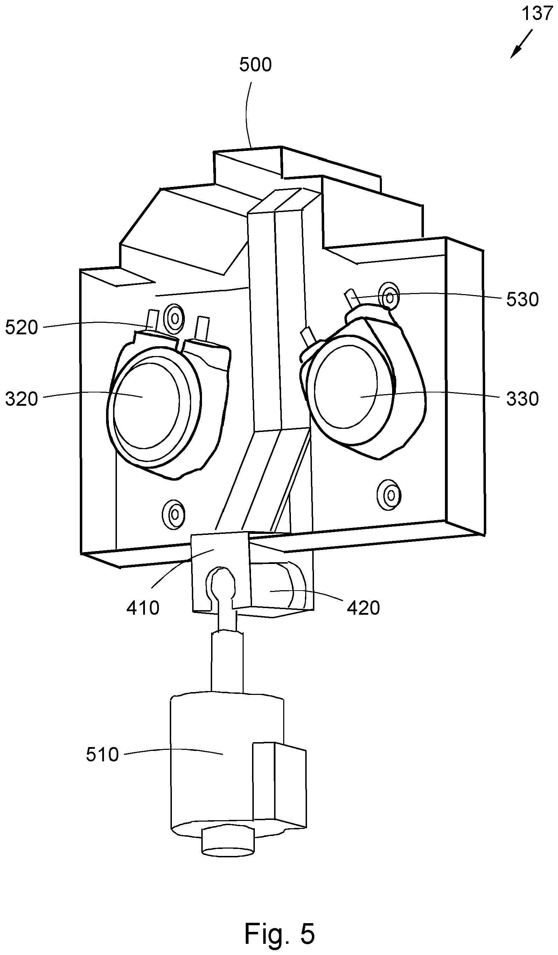

Figure 1 and Figure 2A depict general overviews of the environment in which the milk analysis apparatus 120, the cassette 130, the liquid container, and the dosing module 137 according to the provided solution is intended to operate, without going too much into details, in order for the reader to get a rough overview. Sublime examples of details of the tape may be studied in Figure 2B. Figure 3A illustrates a particular aspect of the provided solution and interaction between various modules of the milk analysis apparatus 120 while Figure 3B illustrates another view of the cassette 130. Figure 4 and Figure 5 disclose details of an embodiment of the dosing module 137.

**Figure 2A and 2B** illustrates a scenario illustrating a milk analysis apparatus 120, a cassette 130, a liquid container 135 and a dosing module 137 interacting with each other and with the milk extracting arrangement 110, according to an embodiment.

The milk analysis apparatus 120 may comprise electronics and equipment such as e.g. a camera, a tube element for attachment to the milk extracting arrangement 110, a motor, a communication unit, etc., to be used for determining a biometric value of a milk sample received from an animal 100. In some embodiments, one or several pumps and tubings are comprised in the dosing module 137. The pump/s is configured to act on the tube element for advancing the milk sample from the milk extracting arrangement 110 through the tube element to reach the needle; or the mixing chamber of the needle. The mixing chamber may alternatively be external to the needle. The tube elements are configured to receive the milk sample of the animal 100 via a milk extracting arrangement 110 and provide the milk sample to a needle, i.e. the needle comprised in the dosing module 137.

In the illustrated embodiment, the dosing module 137 may comprise a needle for applying the milk sample to a milk analysis unit 260a, 260b, 260c on a tape 250 in the cassette 130 through an opening in the cassette 130. The camera may then align the needle with the milk analysis unit 260a, 260b, 260c on the tape 250 of the cassette 130, in an embodiment. The milk analysis units 260a, 260b, 260c may not necessarily be kept on a tape 250, but other similar solutions may be applied wherein the milk analysis units 260a, 260b, 260c may be maintained on another similar substrate.

The dosing module 137 may also comprise a liquid evacuator or drainage, which may collect liquid that has been output by the needle. The liquid, when comprising merely milk, may be returned back to the milk line/ milk extracting arrangement 110 in some embodiments. In other embodiments, when the milk has been mixed with diluent, the liquid may be conveyed away from the cassette 130 in order not to soak or contaminate other, unused, milk analysis units 260a, 260b, 260c of the tape 250 on the cassette 130.

The camera may capture an image of the milk analysis unit 260a, 260b, 260c of the carrier tape 250 through the opening, and based on these images, a cassette external motor may adjust the tape 250 for positioning a new milk analysis unit 260a, 260b, 260c, on which a new test is to be made, in relation to the needle.

The milk analysis apparatus 120 may also comprise a communication device which may communicate via a wired or wireless communication interface with an output unit, a database, a communication device of a farmer, etc.

The control unit may be configured to determine a biomarker value of the milk sample of the animal 100, based on an analysis of the image, captured by the camera. The control unit may be comprised in the milk analysis apparatus 120 in some embodiments; or be external to the milk analysis apparatus 120.

A database may store measured biometric values of the animal 100, associated with an identity reference of the animal 100 and/ or a time stamp of the measurement. Other measurements and/ or data related to the animal 100 may also be stored in the database, such as milk yield, e.g. measured by the milk flow meter, activity, breed, parity, rumination, lactation, resting, feed intake, energy balance, Days In Milk, milk production, age and possibly other similar animal status related parameters.

When a deviation, exceeding a first threshold limit, is detected between the outcomes of the biomarker measurement and the corresponding reference value, an alert may be outputted to the farmer or other responsible person. The alert may comprise e.g. visual information, an audio message, a tactile signal or a combination thereof, encouraging the farmer to further investigate the reasons for the detected deviation in result. In case a plurality of people is working with the herd, a broadcast may be made to the plurality of farmers and their respective associated output units, in some embodiments.

**Figure 3A** illustrates the interaction between the milk analysis apparatus 120, the cassette 130, the liquid container 135 and/ or the dosing module 137.

The milk analysis apparatus 120 comprises a first wireless communication device 210. The first wireless communication device 210 is configured for wireless communication with a second wireless communication device 220 comprised in the dosing module 137. The wireless communication between the devices 210, 220 may be made by e.g. NFC communication, Bluetooth, Radio-Frequency Identification (RFID) or other similar short-range wireless communication.

The milk analysis apparatus 120 comprises a control unit 240 while the dosing module 137 comprises a memory device 230 for storage of data. The control unit 240 may optionally be situated outside of the milk analysis apparatus 120 yet being in communicational contact with the first wireless communication device 210.

The control unit 240 of the milk analysis apparatus 120 thereby obtains information from the dosing module 137, which is stored in the memory device 230 of the dosing module 137 and/ or provide information to the memory device 230 of the dosing module 137, for storage therein.

When producing or rehabilitating the dosing module 137, a representation of the production time may be stored in the memory device 230 of the dosing module 137, possibly associated with a time code and/ or a pump and or tubing quality identifying code.

The memory device 230 of the dosing module 137 may in some embodiments comprise an indicator of virginity, which is stored in the memory device 230 during production or restauration of the dosing module 137, when the pump of the dosing module 137 is unused. Further, the memory device 230 may provide the indicator of virginity to the control unit 240 of the milk analysis apparatus 120, via the second wireless communication device 220, when the pump is used for the first time.

The control unit 240 of the milk analysis apparatus 120 may, upon receiving the indicator of virginity from the memory device 230 of the dosing module 137, determine a moment in time of the first-time usage of the pumps in the dosing module 137 and provide this moment in time to the memory device 230, via the second wireless communication device 220, for storage therein.

Hereby a time reference is provided, enabling a later made life time estimation of the dosing module 137 by extracting the moment in time of first-time usage of the dosing module 137 from the memory device 230, determining the current moment in time, calculating an operational time of the dosing module 137 based on the first-time usage and the current moment in time. This calculated operational time of the dosing module 137 may then be compared with a time threshold limit of the dosing module 137.

Different dosing modules 137 may comprise tubings and/ or pumps having different quality and thereby also differently length of expected lifetime of the components of the dosing module 137.

A control unit 240 of the milk analysis apparatus 120 is in communicational connection with the first wireless communication device 210. Thereby, the control unit 240 obtains information stored in the memory device 230 of the dosing module 137 and/ or provides information via the wireless communication devices 210, 220 to the memory device 230 of the dosing module 137 for storage therein.

The control unit 240 may also obtain information from other entities comprised in or associated with the milk analysis apparatus 120. The control unit 240 may for example obtain a signal associated with consumed liquid amount of the liquid container 135, during a liquid extraction session from a liquid volume determination device, such as e.g. a pump in the dosing module, or a flow sensor configured to estimate amount of liquid that has passed the tubings.

The control unit 240 may determine a measurement relating to usage of the pump of the dosing module 137. The control unit 240 may then transmit information concerning the determined measurement to the memory device 230 of the dosing module 137, via the first wireless communication device 210, for storage in the memory device 230 of the dosing module 137.

It hereby becomes possible to continuously keep track of the amount of usage of the pump of the dosing module 137; either the total running time of the pump, or the total pumped volume of liquid (i.e. during the lifetime of the dosing module 137); also in case the dosing module 137 is demounted and inserted into another milk analysis apparatus 120.

The control unit 240 may also detect when the pump of the dosing module 137 is used for the first time and determine a moment in time when the first-time usage of the pump is detected. Also, this information may be transmitted to the determined moment in time to the memory device 230 of the dosing module 137, via the first wireless communication device 210, for storage in the memory device 230 of the dosing module 137.

Further, in some embodiments, the control unit 240 may determine a current moment in time. Also, the control unit 240 may check whether the past time between the stored moment in time of the memory device 230 of the dosing module 137, associated with the first-time usage of the pump, and the determined current moment in time exceeds a predetermined time limit. In case the predetermined time limit is exceeded, usage of the dosing module 137 may be discontinued by the control unit 240.

The measurement relating to usage of the pump may comprise a time gap of inactivity of the pump, e.g. due to a malfunction of the milk analysis apparatus 120, which then is sent to service; electricity shortage; or other similar reason. The control unit 240 may then obtain a time value indicating when the pump was last used, from the memory device 230 of the dosing module 137, via the first wireless communication device 210. The control unit 240 may also determine a current moment in time. In addition, the control unit 240 may also check whether the past time between the current moment in time and the time value of the last pump usage exceeds a predetermined inactivity time limit. The control unit 240 may then discontinue usage of the dosing module 137 when the predetermined inactivity time limit is exceeded.

It is hereby avoided that a dosing module 137 which has been inactive for too long time (i.e. a time period exceeding the predetermined time limit) is inserted and used in a milk analysis apparatus 120, as the period of inactivity may have caused deterioration of tubings and/ or pumps of the dosing module 137.

The measurement relating to usage of the pump may in some embodiments comprise pumped amount of liquid by the pump. The control unit 240 may then obtain a value indicating amount of pumped liquid made by the pump, from the memory device 230 of the dosing module 137, via the first wireless communication device 210 in case the pump has been previously used. Also, the control unit 240 may determine amount of pumped liquid by the pump during a test session. Furthermore, the control unit 240 may calculate an amount of totally pumped liquid by the pump by adding the amount of liquid pumped during the test session to the obtained amount of previously pumped liquid. A transmission may then be made, transmitting the calculated amount of totally pumped liquid by the pump to the memory device 230 of the dosing module 137, via the first wireless communication device 210, for storage in the memory device 230 of the dosing module 137.

The control unit 240 may also check whether the calculated amount of totally pumped liquid by the pump exceeds a predetermined volume limit by comparing the amount of totally pumped liquid of the pump with the predetermined volume limit. Also, the control unit 240 may discontinue usage of the dosing module 137 when the predetermined volume limit is exceeded.

It is thereby avoided that the dosing module 137 is used excessively much. The control unit 240 may generate and store a blocking sign prohibiting further usage of the dosing module 137, via communication through the first wireless communication device 210 and the second wireless communication device 220, in the memory device 230 of the dosing module 137.

In yet some embodiments wherein the measurement relating to usage of the pump comprises running time of the pump, the control unit 240 may be configured to estimate a total running time of the pump, aggregated during the whole lifetime of the dosing module 137 since its first-time usage. The control unit 240 may be configured to compare the total running time of the pump with a running time threshold limit. Also, the control unit 240 may be configured to discontinue usage of the dosing module 137 when the running time threshold limit is exceeded. The control unit 240 may generate and store a blocking sign prohibiting further usage of the dosing module 137, via communication through the first wireless communication device 210 and the second wireless communication device 220, in the memory device 230 of the dosing module 137.

It is thereby avoided that the dosing module 137 is used excessively much without exchanging it for a new one. Thereby, functionality failure of the milk analysis apparatus 120 due to deteriorated tubings/ pumps is avoided.

The control unit 240 may furthermore be configured to output an alert, encouraging an agricultural manager to change the dosing module 137 when any of the predetermined time limit, the predetermined inactivity time limit, the predetermined volume limit, and/ or the running time threshold limit is exceeded, via an output device 310 of the agricultural manager, i.e. farmer/ operator.

The output device 310 may for example be e.g. a cellular mobile telephone, a stationary or portable computing device, a computer tablet, a display, a pair of intelligent glasses, a smart contact lens, an augmented reality device, a smart watch or similar device having a user interface and wireless communication ability.

By alerting the agricultural manager/ farmer, he/ she becomes aware of the situation with the dosing module 137 and is reminded to exchange the dosing module 137 of the particular milk analysis apparatus 120, thereby relieving him/ her from the tedious work of continuously monitoring the respective status of the dosing modules 137 of different milk analysis apparatus 120 at the farm.

In yet some embodiments, the control unit 240 may be configured to activate the first wireless communication device 210 when starting a liquid extraction session with the pump 320, 330. Also, the control unit 240 may be configured to deactivate the first wireless communication device 210 when the information concerning the determined measurement to the memory device 230 of the dosing module 137 has been transmitted.

It thereby becomes possible to keep the wireless communication devices 210, 220 active for as brief period of time as possible. Thereby, transmission disturbance on other wireless communication devices close-by is avoided or at least reduced. Also, energy is saved.

**Figure 3B** illustrates the cassette 130 with the tape 250 comprising milk analysis units 260a, 260b, 260c on the tape 250.

**Figure 4** illustrates the dosing module 137 as regarded from an exterior side, comprising a first cover section 400, or cover shield, of the dosing module 137. The first cover section 400 may be made of plastic such as e.g. polyamide, polycarbonate, polyethylene, polypropylene, polyvinylchloride, etc., and may cover the interior of the dosing module 137. Thereby, the pumps and tubings are protected from dust, dirt and pollution of the farm.

In Figure 4, a position adjustment mechanism 410 may be observed, having a ball joint 420, to be connected to a linear motor.

The first cover section 400 may also comprise fastening means 430a, 430b, 430c, 430d, e.g. in form of a snap fit arrangement, magnetics, screw joints, etc., arranged to attach the dosing module 137 onto the milk analysis apparatus 120.

**Figure 5** illustrates a second cover section 500, illustrating the interior of the dosing module 137, onto which the first cover section 400 is to be mounted to complete the dosing module 137.

The dosing module 137 comprises a milk insertion connection 520, arranged to receive milk from a milking equipment. Further, the dosing module 137 comprises a first pump 320, configured to provide milk from the milk insertion connection 520 to the needle of the dosing module 137.

In some embodiments, the dosing module 137 also may comprise a diluent insertion connection 530, arranged to receive diluent. Further, the dosing module 137 may comprise a mixing chamber, arranged to receive milk from the liquid insertion connection 520 and diluent from the diluent insertion connection 530. The dosing module 137 may also comprise a second pump 330, configured to provide diluent from the diluent insertion connection 530 to the mixing chamber.

The first pump 320 may be driven by the first pump motor of the milk analysis apparatus 120, while the optional second pump 330 may be driven by the second pump motor of the milk analysis apparatus 120.

The first pump 320 and/ or the second pump 330 may comprise a positive displacement pump, such as a peristaltic pump, which also may be referred to as a hose pump, a tube pump and/ or a microfluidic pump.

The fluid such as milk or diluent is comprised within a flexible tube fitted inside a respective circular pump casing of the first pump 320 and/ or the second pump 330. A rotor with a number of "rollers", "shoes", "wipers", or "lobes" attached to the external circumference of the rotor compresses the flexible tube. As the rotor turns, the part of the tube under compression is pinched closed thus forcing the fluid to be pumped to move through the tube. Additionally, as the tube opens to its natural state after the passing of the cam ("restitution" or "resilience") fluid flow is induced to the pump 320, 330. This process may be referred to as peristalsis. Typically, there may be two or more rollers, or wipers, occluding the tube, trapping between them a body of fluid. The body of fluid is then transported, at ambient pressure, toward the pump outlet. The pumps 320, 330 may run continuously in some embodiments, or they may be indexed through partial revolutions to deliver smaller amounts of fluid, such as e.g. some millilitres.

An advantage with using peristaltic pumps 320, 330 is that contamination is avoided, as the only part of the pump 320, 330 in contact with the milk being pumped is the interior of the tube, it is easy to sterilise and clean the inside surfaces of the pump 320, 330. Another advantage is that the pumps 320, 330 require only very low, if any, maintenance as the pumps 320, 330 lack valves, seals and glands, etc.

The position adjustment mechanism 410 may be configured to cooperate with a linear motor 510 in some embodiments, for adjusting the position adjustment mechanism 410, adjusting the position of the needle. The movement may be a linear movement, or a lever movement. The linear motor 510 may be comprised in the milk analysis apparatus 120, for example.

The embodiments, or parts thereof, illustrated in Figure 1, Figure 2A, Figure 2B, Figure 3A, Figure 3B, Figure 4, and/ or Figure 5 may with advantage be combined with each other for achieving further benefits.

The terminology used in the description of the embodiments as illustrated in the accompanying drawings is not intended to be limiting of the described control unit 240, the dosing module 137, liquid container, milk analysis apparatus 120 and/ or system 10. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware, but may also be distributed in other forms such as via Internet or other wired or wireless communication system.

## Claims

1. A system (10) comprising:
a control unit (240);
a dosing module (137) comprising a pump (320, 330);
a milk analysis apparatus (120); and
a milk extracting arrangement (110), operating in conjunction with the milk analysis apparatus (120); **characterised in that** the dosing module (137) comprises a memory device (230) and a second wireless communication device (220), which dosing module (137) is insertable into the milk analysis apparatus (120), for information exchange with the control unit (240) of the milk analysis apparatus (120) via a first wireless communication device (210) of the milk analysis apparatus (120); wherein the memory device (230) is configured to:
receive information concerning a measurement relating to usage of the pump (320, 330) from the control unit (240) of the milk analysis apparatus (120), via the second wireless communication device (220); and
store the received information; wherein
the milk analysis apparatus (120) comprises the first wireless communication device (210), for communication with the memory device (230) of the dosing module (137), via the second wireless communication device (220) comprised in the dosing module (137), and wherein the control unit (240) is configured to:
determine a measurement relating to usage of the pump (320, 330); and
transmit information concerning the determined measurement to the memory device (230) of the dosing module (137), via the first wireless communication device (210), for storage in the memory device (230) of the dosing module (137).

2. The system (10) according to claim 1, wherein the measurement relating to usage of the pump (320, 330) comprises usage time and the control unit (240) is configured to:
detect when the pump (320, 330) of the dosing module (137) is used for the first time;
determine a moment in time when the first-time usage of the pump (320, 330) is detected; and
transmit the determined moment in time to the memory device (230) of the dosing module (137), via the first wireless communication device (210), for storage in the memory device (230) of the dosing module (137).

3. The system (10) according to any one of claim 1 or claim 2, wherein the control unit (240) is configured to:
determine a current moment in time;
check whether the past time between the stored moment in time of the memory device (230) of the dosing module (137), associated with the first-time usage of the pump (320, 330), and the determined current moment in time exceeds a predetermined time limit; and
discontinue usage of the dosing module (137) when the predetermined time limit is exceeded.

4. The system (10) according to any one of claims 1-3, wherein the measurement relating to usage of the pump (320, 330) comprises a time gap of inactivity of the pump (320, 330); and the control unit (240) is configured to:
obtain a time value indicating when the pump (320, 330) was last used, from the memory device (230) of the dosing module (137), via the first wireless communication device (210);
determine a current moment in time;
check whether the past time between the current moment in time and the time value of the last pump usage exceeds a predetermined inactivity time limit; and
discontinue usage of the dosing module (137) when the predetermined inactivity time limit is exceeded.

5. The system (10) according to any one of claims 1-4, wherein the measurement relating to usage of the pump (320, 330) comprises pumped amount of liquid by the pump (320, 330); and the control unit (240) is configured to:
obtain a value indicating amount of pumped liquid made by the pump (320, 330), from the memory device (230) of the dosing module (137), via the first wireless communication device (210) in case the pump (320, 330) has been previously used;
determine amount of pumped liquid by the pump (320, 330) during a test session;
calculate an amount of totally pumped liquid by the pump (320, 330) by adding the amount of liquid pumped during the test session to the obtained amount of previously pumped liquid;
transmit the calculated amount of totally pumped liquid by the pump (320, 330) to the memory device (230) of the dosing module (137), via the first wireless communication device (210), for storage in the memory device (230) of the dosing module (137);
check whether the calculated amount of totally pumped liquid by the pump (320, 330) exceeds a predetermined volume limit; and
discontinue usage of the dosing module (137) when the predetermined volume limit is exceeded.

6. The system (10) according to any one of claims 1-5, wherein the measurement relating to usage of the pump (320, 330) comprises running time of the pump (320, 330); and the control unit (240) is configured to:
estimate a total running time of the pump (320, 330);
compare the total running time of the pump (320, 330) with a running time threshold limit; and
discontinue usage of the dosing module (137) when the running time threshold limit is exceeded.

7. The system (10) according to any one of claims 1-6, wherein the control unit (240) is configured to:
output an alert, encouraging an agricultural manager to change the dosing module (137) when any of the predetermined time limit, the predetermined inactivity time limit, the predetermined volume limit, and/ or the running time threshold limit is exceeded, via an output device (310).

8. The system (10) according to any one of claims 1-7, wherein the control unit (240) is configured to:
activate the first wireless communication device (210) when starting a liquid extraction session with the pump (320, 330); and
deactivate the first wireless communication device (210) when the information concerning the determined measurement to the memory device (230) of the dosing module (137) has been transmitted.

9. The system (10) according to claim 1, wherein the memory device (230) comprises an indicator of virginity when the pump (320, 330) of the dosing module (137) is unused; and wherein the memory device (230) is configured to:
provide the indicator of virginity to the control unit (240) of the milk analysis apparatus (120), via the second wireless communication device (220), when the pump (320, 330) is used for the first time.

10. The system (10) according to any one of claim 1 or claim 9, wherein the memory device (230) is configured to:
receive information concerning a moment in time of a usage of the pump (320, 330) from the control unit (240) of the milk analysis apparatus (120), via the second wireless communication device (220);
store the received information; and
provide stored information concerning a moment in time of a first-time usage of the pump (320, 330) to the control unit (240) of the milk analysis apparatus (120), via the second wireless communication device (220).

11. The system (10) according to any one of claims 1 or 9-10, wherein the memory device (230) is configured to:
provide stored information concerning a time value indicating when the pump (320, 330) was last used, to the control unit (240) of the milk analysis apparatus (120), via the second wireless communication device (220).

12. The system (10) according to any one of claims 9-11, wherein the memory device (230) is configured to:
provide a value indicating amount of pumped liquid made liquid by the pump (320, 330), to the control unit (240) of the milk analysis apparatus (120), via the second wireless communication device (220);
obtain an updated value indicating amount of pumped liquid made liquid by the pump (320, 330), from the control unit (240) of the milk analysis apparatus (120), via the second wireless communication device (220); and
store the received information.

13. The system (10) according to any one of claims 9-12, wherein the memory device (230) is configured to:
receive a blocking sign prohibiting further usage of the dosing module (137) from the control unit (240) via the second wireless communication device (220); and
store the received blocking sign, thereby prohibiting further usage of the dosing module (137).

## Patentansprüche

1. System (10), umfassend:
eine Steuereinheit (240);
ein Dosiermodul (137) umfassend eine Pumpe (320, 330);
eine Milchanalyseeinrichtung (120); und
eine Milchextraktionsanordnung (110), die in Verbindung mit der Milchanalyseeinrichtung (120) in Betrieb ist; **dadurch gekennzeichnet, dass**
das Dosiermodul (137) eine Speichervorrichtung (230) und eine zweite Drahtloskommunikationsvorrichtung (220) umfasst, wobei das Dosiermodul (137) in die Milchanalyseeinrichtung (120) für einen Informationsaustausch mit der Steuereinheit (240) der Milchanalyseeinrichtung (120) über eine erste Drahtloskommunikationsvorrichtung (210) der Milchanalyseeinrichtung (120) einsetzbar ist; wobei die Speichervorrichtung (230) konfiguriert ist zum:
Empfangen von Informationen betreffend eine Messung bezüglich einer Verwendung der Pumpe (320, 330) von der Steuereinheit (240) der Milchanalyseeinrichtung (120) über die zweite Drahtloskommunikationsvorrichtung (220); und
Speichern der empfangenen Informationen; wobei
die Milchanalyseeinrichtung (120) die erste Drahtloskommunikationsvorrichtung (210) für eine Kommunikation mit der Speichervorrichtung (230) des Dosiermoduls (137) über die zweite Drahtloskommunikationsvorrichtung (220), die in dem Dosiermodul (137) enthalten ist, umfasst und wobei die Steuereinheit (240) konfiguriert ist zum:
Bestimmen einer Messung bezüglich der Verwendung der Pumpe (320, 330); und
Übertragen von Informationen betreffend die bestimmte Messung an die Speichervorrichtung (230) des Dosiermoduls (137) über die erste Drahtloskommunikationsvorrichtung (210) für eine Speicherung in der Speichervorrichtung (230) des Dosiermoduls (137).

2. System (10) nach Anspruch 1, wobei die Messung bezüglich der Verwendung der Pumpe (320, 330) eine Verwendungszeit umfasst und die Steuereinheit (240) konfiguriert ist zum:
Erkennen, wenn die Pumpe (320, 330) des Dosiermoduls (137) zum ersten Mal verwendet wird;
Bestimmen eines Zeitpunkts, wenn die erstmalige Verwendung der Pumpe (320, 330) erkannt wird; und
Übertragen des bestimmten Zeitpunkts an die Speichervorrichtung (230) des Dosiermoduls (137) über die erste Drahtloskommunikationsvorrichtung (210) für die Speicherung in der Speichervorrichtung (230) des Dosiermoduls (137).

3. System (10) nach einem der Ansprüche 1 oder 2, wobei die Steuereinheit (240) konfiguriert ist zum:
Bestimmen eines aktuellen Zeitpunkts;
Prüfen, ob die vergangene Zeit zwischen dem gespeicherten Zeitpunkt der Speichervorrichtung (230) des Dosiermoduls (137), der der erstmaligen Verwendung der Pumpe (320, 330) zugeordnet ist, und dem bestimmten aktuellen Zeitpunkt eine vorbestimmte Zeitgrenze überschreitet; und
Unterbrechen der Verwendung des Dosiermoduls (137), wenn die vorbestimmte Zeitgrenze überschritten wird.

4. System (10) nach einem der Ansprüche 1 bis 3, wobei die Messung bezüglich der Verwendung der Pumpe (320, 330) eine Zeitlücke einer Inaktivität der Pumpe (320, 330) umfasst; und die Steuereinheit (240) konfiguriert ist zum:
Erhalten eines Zeitwerts, der anzeigt, wann die Pumpe (320, 330) zuletzt verwendet wurde, von der Speichervorrichtung (230) des Dosiermoduls (137) über die erste Drahtloskommunikationsvorrichtung (210);
Bestimmen eines aktuellen Zeitpunkts;
Prüfen, ob die vergangene Zeit zwischen dem aktuellen Zeitpunkt und dem Zeitwert der letzten Pumpenverwendung eine vorbestimmte Inaktivitätszeitgrenze überschreitet; und
Unterbrechen der Verwendung des Dosiermoduls (137), wenn die vorbestimmte Inaktivitätszeitgrenze überschritten wird.

5. System (10) nach einem der Ansprüche 1 bis 4, wobei die Messung bezüglich der Verwendung der Pumpe (320, 330) eine gepumpte Menge an Flüssigkeit durch die Pumpe (320, 330) umfasst; und die Steuereinheit (240) konfiguriert ist zum:
Erhalten eines Werts, der eine Menge an gepumpter Flüssigkeit, die durch die Pumpe (320, 330) gemacht wird, anzeigt, von der Speichervorrichtung (230) des Dosiermoduls (137) über die erste Drahtloskommunikationsvorrichtung (210) falls die Pumpe (320, 330) zuvor verwendet wurde;
Bestimmen der Menge an gepumpter Flüssigkeit durch die Pumpe (320, 330) während einer Testsitzung;
Berechnen einer Menge an insgesamt gepumpter Flüssigkeit durch die Pumpe (320, 330) durch Addieren der Menge an Flüssigkeit, die während der Testsitzung gepumpt wird, zu der erhaltenen Menge an zuvor gepumpter Flüssigkeit;
Übertragen der berechneten Menge an insgesamt gepumpter Flüssigkeit durch die Pumpe (320, 330) an die Speichervorrichtung (230) des Dosiermoduls (137) über die erste Drahtloskommunikationsvorrichtung (210) für die Speicherung in der Speichervorrichtung (230) des Dosiermoduls (137);
Prüfen, ob die berechnete Menge an insgesamt gepumpter Flüssigkeit durch die Pumpe (320, 330) eine vorbestimmte Volumengrenze überschreitet; und
Unterbrechen der Verwendung des Dosiermoduls (137), wenn die vorbestimmte Volumengrenze überschritten wird.

6. System (10) nach einem der Ansprüche 1 bis 5, wobei die Messung bezüglich der Verwendung der Pumpe (320, 330) eine Laufzeit der Pumpe (320, 330) umfasst; und die Steuereinheit (240) konfiguriert ist zum:
Schätzen einer Gesamtlaufzeit der Pumpe (320, 330);
Vergleichen der Gesamtlaufzeit der Pumpe (320, 330) mit einer Laufzeitschwellengrenze; und
Unterbrechen der Verwendung des Dosiermoduls (137), wenn die vorbestimmte Laufzeitschwellengrenze überschritten wird.

7. System (10) nach einem der Ansprüche 1 bis 6, wobei die Steuereinheit (240) konfiguriert ist zum:
Ausgeben einer Warnung, die einen landwirtschaftlichen Manager dazu anregt, das Dosiermodul (137) zu wechseln, wenn eine beliebige der vorbestimmten Zeitgrenze, der vorbestimmten Inaktivitätszeitgrenze, der vorbestimmten Volumengrenze und/oder der Laufzeitschwellengrenze überschritten wird, über eine Ausgabevorrichtung (310).

8. System (10) nach einem der Ansprüche 1 bis 7, wobei die Steuereinheit (240) konfiguriert ist zum:
Aktivieren der ersten Drahtloskommunikationsvorrichtung (210), wenn eine Flüssigkeitsextraktionssitzung mit der Pumpe (320, 330) gestartet wird; und
Deaktivieren der ersten Drahtloskommunikationsvorrichtung (210), wenn die Information betreffend die bestimmte Messung an die Speichereinrichtung (230) des Dosiermoduls (137) übertragen wurde.

9. System (10) nach Anspruch 1, wobei die Speichervorrichtung (230) eine Anzeige einer Unberührtheit umfasst, wenn die Pumpe (320, 330) des Dosiermoduls (137) nicht verwendet ist; und wobei die Speichervorrichtung (230) konfiguriert ist zum:
Bereitstellen der Anzeige der Unberührtheit an die Steuereinheit (240) der Milchanalyseeinrichtung (120) über die zweite Drahtloskommunikationsvorrichtung (220), wenn die Pumpe (320, 330) zum ersten Mal verwendet wird.

10. System (10) nach einem der Ansprüche 1 oder 9, wobei die Speichervorrichtung (230) konfiguriert ist zum:
Empfangen von Informationen betreffend einen Zeitpunkt einer Verwendung der Pumpe (320, 330) von der Steuereinheit (240) der Milchanalyseeinrichtung (120) über die zweite Drahtloskommunikationsvorrichtung (220);
Speichern der empfangenen Informationen; und
Bereitstellen gespeicherter Informationen betreffend einen Zeitpunkt einer erstmaligen Verwendung der Pumpe (320, 330) an die Steuereinheit (240) der Milchanalyseeinrichtung (120) über die zweite Drahtloskommunikationsvorrichtung (220).

11. System (10) nach einem der Ansprüche 1 oder 9 bis 10, wobei die Speichervorrichtung (230) konfiguriert ist zum:
Bereitstellen gespeicherter Informationen betreffend einen Zeitwert, der anzeigt, wann die Pumpe (320, 330) zuletzt verwendet wurde, an die Steuereinheit (240) der Milchanalyseeinrichtung (120) über die zweite Drahtloskommunikationsvorrichtung (220).

12. System (10) nach einem der Ansprüche 9 bis 11, wobei die Speichervorrichtung (230) konfiguriert ist zum:
Bereitstellen eines Werts, der die Menge an gepumpter Flüssigkeit, die durch die Pumpe (320, 330) flüssig gemacht wird, anzeigt, an die Steuereinheit (240) der Milchanalyseeinrichtung (120) über die zweite Drahtloskommunikationsvorrichtung (220);
Erhalten eines aktualisierten Werts, der die Menge an gepumpter Flüssigkeit, die durch die Pumpe (320, 330) flüssig gemacht wird, anzeigt, von der Steuereinheit (240) der Milchanalyseeinrichtung (120) über die zweite Drahtloskommunikationsvorrichtung (220); und
Speichern der empfangenen Informationen.

13. System (10) nach einem der Ansprüche 9 bis 12, wobei die Speichervorrichtung (230) konfiguriert ist zum:
Empfangen eines Sperrzeichens, das eine weitere Verwendung des Dosiermoduls (137) verhindert, von der Steuereinheit (240) über die zweite Drahtloskommunikationsvorrichtung (220); und
Speichern des empfangenen Sperrzeichens und dadurch Verhindern der weiteren Verwendung des Dosiermoduls (137).

## Revendications

1. Système (10) comprenant :
une unité de commande (240) ;
un module de dosage (137) comprenant une pompe (320, 330) ;
un appareil d'analyse de lait (120) ; et
un agencement d'extraction de lait (110), fonctionnant conjointement avec l'appareil d'analyse de lait (120) ; **caractérisé en ce que**
le module de dosage (137) comprend un dispositif de mémoire (230) et un second dispositif de communication sans fil (220), lequel module de dosage (137) peut être inséré dans l'appareil d'analyse de lait (120), pour l'échange d'informations avec l'unité de commande (240) de l'appareil d'analyse de lait (120) par l'intermédiaire d'un premier dispositif de communication sans fil (210) de l'appareil d'analyse de lait (120) ; dans lequel le dispositif de mémoire (230) est configuré pour :
recevoir de l'unité de commande (240) de l'appareil d'analyse de lait (120), par l'intermédiaire du second dispositif de communication sans fil (220), des informations concernant une mesure relative à l'utilisation de la pompe (320, 330) ; et
stocker les informations reçues ; dans lequel
l'appareil d'analyse de lait (120) comprend le premier dispositif de communication sans fil (210), pour la communication avec le dispositif de mémoire (230) du module de dosage (137), par l'intermédiaire du second dispositif de communication sans fil (220) compris dans le module de dosage (137), et dans lequel l'unité de commande (240) est configurée pour :
déterminer une mesure relative à l'utilisation de la pompe (320, 330) ; et
transmettre des informations concernant la mesure déterminée au dispositif de mémoire (230) du module de dosage (137), par l'intermédiaire du premier dispositif de communication sans fil (210), pour stockage dans le dispositif de mémoire (230) du module de dosage (137).

2. Système (10) selon la revendication 1, dans lequel la mesure relative à l'utilisation de la pompe (320, 330) comprend un temps d'utilisation et l'unité de commande (240) est configurée pour :
détecter quand la pompe (320, 330) du module de dosage (137) est utilisée pour la première fois ;
déterminer un moment dans le temps où l'utilisation première de la pompe (320, 330) est détectée ; et
transmettre le moment dans le temps déterminé au dispositif de mémoire (230) du module de dosage (137), par l'intermédiaire du premier dispositif de communication sans fil (210), pour stockage dans le dispositif de mémoire (230) du module de dosage (137).

3. Système (10) selon l'une quelconque de la revendication 1 ou de la revendication 2, dans lequel l'unité de commande
(240) est configurée pour :
déterminer un moment actuel dans le temps ;
vérifier si la différence de temps entre le moment dans le temps stocké du dispositif de mémoire (230) du module de dosage (137), associé à l'utilisation première de la pompe (320, 330), et le moment actuel dans le temps déterminé dépasse une limite de temps prédéterminée ; et
cesser l'utilisation du module de dosage (137) lorsque la limite de temps prédéterminée est dépassée.

4. Système (10) selon l'une quelconque des revendications 1 à 3, dans lequel la mesure relative à l'utilisation de la pompe (320, 330) comprend un intervalle de temps d'inactivité de la pompe (320, 330) ; et l'unité de commande (240) est configurée pour :
obtenir à partir du dispositif de mémoire (230) du module de dosage (137), par l'intermédiaire du premier dispositif de communication sans fil (210), une valeur de temps indiquant quand la pompe (320, 330) a été utilisée en dernier lieu ;
déterminer un moment actuel dans le temps ;
vérifier si la différence de temps entre le moment actuel dans le temps et la valeur de temps de la dernière utilisation de la pompe dépasse une limite de temps d'inactivité prédéterminée ; et
cesser l'utilisation du module de dosage (137) lorsque la limite de temps d'inactivité prédéterminée est dépassée.

5. Système (10) selon l'une quelconque des revendications 1 à 4, dans lequel la mesure relative à l'utilisation de la pompe (320, 330) comprend une quantité de liquide pompée par la pompe (320, 330) ; et l'unité de commande (240) est configurée pour :
obtenir à partir du dispositif de mémoire (230) du module de dosage (137), par l'intermédiaire du premier dispositif de communication sans fil (210) dans le cas où la pompe (320, 330) a été précédemment utilisée, une valeur indiquant la quantité de liquide pompé réalisée par la pompe (320, 330) ;
déterminer la quantité de liquide pompé par la pompe (320, 330) pendant une session de test ;
calculer une quantité de liquide totalement pompé par la pompe (320, 330) par l'ajout de la quantité de liquide pompé pendant la session de test à la quantité obtenue de liquide précédemment pompé ;
transmettre la quantité calculée de liquide totalement pompé par la pompe (320, 330) au dispositif de mémoire (230) du module de dosage (137), par l'intermédiaire du premier dispositif de communication sans fil (210), pour stockage dans le dispositif de mémoire (230) du module de dosage (137) ;
vérifier si la quantité calculée de liquide totalement pompé par la pompe (320, 330) dépasse une limite de volume prédéterminée ; et
cesser l'utilisation du module de dosage (137) lorsque la limite de volume prédéterminée est dépassée.

6. Système (10) selon l'une quelconque des revendications 1 à 5, dans lequel la mesure relative à l'utilisation de la pompe (320, 330) comprend le temps de marche de la pompe (320, 330) ; et l'unité de commande (240) est configurée pour :
estimer un temps de marche total de la pompe (320, 330) ;
comparer le temps de marche total de la pompe (320, 330) à une limite de seuil de temps de marche ; et
cesser l'utilisation du module de dosage (137) lorsque la limite de seuil de temps de marche est dépassée.

7. Système (10) selon l'une quelconque des revendications 1 à 6, dans lequel l'unité de commande (240) est configurée pour :
sortir une alerte, encourageant un gestionnaire agricole à changer le module de dosage (137) lorsque l'une quelconque de la limite de temps prédéterminée, de la limite de temps d'inactivité prédéterminée, de la limite de volume prédéterminée, et/ou de la limite de seuil de temps de marche est dépassée, par l'intermédiaire d'un dispositif de sortie (310).

8. Système (10) selon l'une quelconque des revendications 1 à 7, dans lequel l'unité de commande (240) est configurée pour :
activer le premier dispositif de communication sans fil (210) lors du démarrage d'une session d'extraction de liquide avec la pompe (320, 330) ; et
désactiver le premier dispositif de communication sans fil (210) lorsque les informations concernant la mesure déterminée ont été transmises au dispositif de mémoire (230) du module de dosage (137).

9. Système (10) selon la revendication 1, dans lequel le dispositif de mémoire (230) comprend un indicateur de virginité lorsque la pompe (320, 330) du module de dosage (137) est inutilisée ; et dans lequel le dispositif de mémoire (230) est configuré pour :
fournir l'indicateur de virginité à l'unité de commande (240) de l'appareil d'analyse de lait (120), par l'intermédiaire du second dispositif de communication sans fil (220), lorsque la pompe (320, 330) est utilisée pour la première fois.

10. Système (10) selon l'une quelconque de la revendication 1 ou de la revendication 9, dans lequel le dispositif de mémoire (230) est configuré pour :
recevoir de l'unité de commande (240) de l'appareil d'analyse de lait (120), par l'intermédiaire du second dispositif de communication sans fil (220), des informations concernant un moment dans le temps d'une utilisation de la pompe (320, 330) ;
stocker les informations reçues ; et
fournir des informations stockées concernant un moment dans le temps d'une utilisation première de la pompe (320, 330) à l'unité de commande (240) de l'appareil d'analyse de lait (120), par l'intermédiaire du second dispositif de communication sans fil (220).

11. Système (10) selon l'une quelconque des revendications 1 ou 9 à 10, dans lequel le dispositif de mémoire (230) est configuré pour :
fournir des informations stockées concernant une valeur de temps indiquant quand la pompe (320, 330) a été utilisée en dernier lieu, à l'unité de commande (240) de l'appareil d'analyse de lait (120), par l'intermédiaire du second dispositif de communication sans fil (220).

12. Système (10) selon l'une quelconque des revendications 9 à 11, dans lequel le dispositif de mémoire (230) est configuré pour :
fournir une valeur indiquant la quantité de liquide pompé rendu liquide par la pompe (320, 330), à l'unité de commande (240) de l'appareil d'analyse de lait (120), par l'intermédiaire du second dispositif de communication sans fil (220) ;
obtenir à partir de l'unité de commande (240) de l'appareil d'analyse de lait (120), par l'intermédiaire du second dispositif de communication sans fil (220), une valeur mise à jour indiquant la quantité de liquide pompé rendu liquide par la pompe (320, 330) ; et
stocker les informations reçues.

13. Système (10) selon l'une quelconque des revendications 9 à 12, dans lequel le dispositif de mémoire (230) est configuré pour :
recevoir de l'unité de commande (240) par l'intermédiaire du second dispositif de communication sans fil (220) un signe de blocage interdisant une utilisation supplémentaire du module de dosage (137) ; et
stocker le signe de blocage reçu, interdisant de ce fait une utilisation supplémentaire du module de dosage (137).
